(19) Europäisches Patentamt
European Patent Office
Office européen des brevets

(11) **EP 3 972 951 B1**

(12) **EUROPÄISCHE PATENTSCHRIFT**

(45) Veröffentlichungstag und Bekanntmachung des Hinweises auf die Patenterteilung:
**03.04.2024   Patentblatt 2024/14**

(21) Anmeldenummer: **20726805.3**

(22) Anmeldetag: **18.05.2020**

(51) Internationale Patentklassifikation (IPC):
**C07C 41/56** *(2006.01)*    **C07C 41/58** *(2006.01)*

(52) Gemeinsame Patentklassifikation (CPC):
(C-Sets verfügbar)
**C07C 41/56; C07C 41/58;** Y02P 20/10        (Forts.)

(86) Internationale Anmeldenummer:
**PCT/EP2020/063774**

(87) Internationale Veröffentlichungsnummer:
**WO 2020/234220 (26.11.2020 Gazette 2020/48)**

(54) **VERFAHREN ZUR HERSTELLUNG VON POLYOXYMETHYLENDIMETHYLETHER**

METHOD FOR PRODUCING POLYOXYMETHYLENE DIMETHYL ETHERS

PROCÉDÉ DE PRODUCTION DE DIMÉTHYLÉTHER DE POLYOXYMÉTHYLÈNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.05.2019   DE 102019207540**

(43) Veröffentlichungstag der Anmeldung:
**30.03.2022   Patentblatt 2022/13**

(73) Patentinhaber: **Fraunhofer-Gesellschaft zur Förderung
der angewandten Forschung e.V.
80686 München (DE)**

(72) Erfinder:
• **OUDA, Mohamed**
**79110 Freiburg (DE)**
• **MANTEI, Franz M.**
**79110 Freiburg (DE)**

• **SCHAADT, Achim**
**79110 Freiburg (DE)**

(74) Vertreter: **Maiwald GmbH
Elisenhof
Elisenstraße 3
80335 München (DE)**

(56) Entgegenhaltungen:
WO-A1-2007/051658          CN-B- 102 372 615
DE-A1-102004 053 839       DE-A1-102016 222 657
US-A1- 2002 007 089

(52) Gemeinsame Patentklassifikation (CPC): (Forts.)

C-Sets
**C07C 41/56, C07C 43/30;**
**C07C 41/58, C07C 43/30**

**Beschreibung**

Die vorliegende Erfindung betrifft ein Verfahren zur Herstellung von Polyoxymethylendimethylether in einer Reaktivdestillationseinheit.

Synthetische Energieträger, welche nicht auf Basis von Rohöl oder Erdgas hergestellt werden, können die Abhängigkeit von fossilen Energiequellen sowie die aus deren Verwendung entstehende Umweltbelastung verringern. Ein Beispiel für einen solchen Energieträger sind Polyoxymethylendimethylether (OMEs). Polyoxymethylendimethylether (OMEs) können aus Kohlenstoffdioxid und Wasser hergestellt werden und weisen, soweit regenerative Energieträger für deren Herstellung eingesetzt werden, bei der Umsetzung als Kraftstoff einen geschlossenen Kohlenstoffdioxidkreislauf auf.

Darüber hinaus bietet die Nutzung der Polyoxymethylendimethylether als Energieträger weitere Vorteile. Polyoxymethylendimethylether weisen keine Kohlenstoff-Kohlenstoff-Bindungen auf und besitzen außerdem einen hohen Anteil an Sauerstoff. Polyoxymethylendimethylether verbrennen rußfrei und schonen damit sowohl den Verbrennungsmotor und nachgeschaltete Filterelemente als auch die Umwelt. Polyoxymethylendimethylether mit drei bis fünf Oxymethylen-Einheiten ($OME_{3-5}$) sind dabei von besonderem Interesse.

Derzeit sind insbesondere zwei Herstellungsverfahren für $OME_{3-5}$ im Einsatz, die auf Polyadditions- oder Polykondensationsreaktionen basieren.

Polyaddition:

$$CH_2O + CH_3O\text{-}(CH_2O)_{n-1}\text{-}CH_{3(l)} \leftrightarrows CH_3O\text{-}(CH_2O)_n\text{-}CH_{3(l)}$$

Polykondensation mit Halbacetalbildung:

$$CH_2O + CH_3OH \leftrightarrows HO\text{-}CH_2O\text{-}CH_{3(l)} \qquad HF$$

$$CH_3OH + HO\text{-}(CH_2O)_1\text{-}CH_{3(l)} \leftrightarrows CH_3O\text{-}(CH_2O)_1\text{-}CH_{3(l)} + H_2O \qquad OME1$$

$$CH_2O + H_2O \leftrightarrows HO\text{-}CH_2O\text{-}H_{(l)} \qquad MG$$

HF: Poly(oxymethylen)hemiformal
MG: Poly(oxymethylen)glycol

Bei der Synthese von Polyoxymethylendimethylether werden als Edukte üblicherweise eine Formaldehydquelle (z.B. Formaldehyd, Trioxan oder Paraformaldehyd) und eine Verbindung für die Methylverkappung (engl.: "methyl capping") wie Methanol, Methylal oder Dimethylether (d.h. eine Verbindung, die eine Hydroxylgruppe in eine Methoxygruppe überführen kann) verwendet.

Eine Übersicht bekannter Herstellungsverfahren für Polyoxymethylendimethylether findet sich in den folgenden Publikationen:

- M. Ouda et al., React. Chem. Eng., 2017, 2, S. 50-59;
- M. Ouda et al., React. Chem. Eng., 3, 2018, S. 676-695
- Z. Wang et al., Applied Energy, 233-234 (2019), S. 599-611.

Die Reaktionen der Edukte zu den Polyoxymethylendimethylethern erfordern üblicherweise die Anwesenheit eines Katalysators, insbesondere die Anwesenheit eines sauren Katalysators. Bekannte Feststoffe, die als Katalysator bei der Polyoxymethylendimethylether-Synthese fungieren können, sind beispielsweise Ionenaustauscherharze, die saure Gruppen aufweisen (d.h. Kationenaustauscherharze), Zeolite, Aluminosilicate, Übergangsmetalloxide (die optional auf einem Trägermaterial vorliegen können) und Graphenoxid. Als flüssige Katalysatoren für die Polyoxymethylendimethylether-Synthese können beispielsweise Mineralsäuren (z.B. Schwefelsäure), organische Säuren (z.B. HCOOH) und saure ionische Flüssigkeiten verwendet werden.

Bei der Polyoxymethylendimethylether-Synthese entsteht durch Kondensationsreaktionen der Reaktionspartner Wasser. Auch ist es möglich, dass bereits die dem Reaktor zugeführten Edukte wasserhaltig sind und so Wasser in das Reaktionssystem eingebracht wird.

Um eine möglichst hohe Ausbeute an Polyoxymethylendimethylether zu realisieren, muss eine Abtrennung des Wassers erfolgen. Ist jedoch Formaldehyd in höherer Konzentration anwesend, erweist sich eine effiziente Wasserabtrennung als sehr schwierig.

Die Wasserabtrennung kann beispielsweise durch Membrane (z.B. durch Pervaporation oder Dampfpermeation) erfolgen. Als Membrane können beispielsweise Polymermembrane wie PVA-Membrane verwendet werden. Untersuchungen des Anmelders haben jedoch gezeigt, dass bei höheren Formaldehydkonzentrationen die Langzeitstabilität von PVA-Membranen nicht ausreichend ist.

Wie oben bereits erwähnt, ist eines der Edukte für die Synthese von Polyoxymethylendimethylether eine Formaldehydquelle. Aufgrund der umweltgefährdenden Eigenschaften des Formaldehyds muss die Reaktionsanlage für die Polyoxymethylendimethylether-Synthese so ausgelegt sein, dass eine Freisetzung des Formaldehyds nicht stattfindet. In diesem Zusammenhang wäre es vorteilhaft, wenn Formaldehyd bei der OME-Synthese möglichst quantitativ umgesetzt wird und eine nachgeschaltete Abtrennung von nicht umgesetztem Formaldehyd entfällt.

EP 3 323 800 A1 beschreibt ein Verfahren zur Herstellung von Polyoxymethylendimethylether, in dem Formaldehyd, Methanol und Wasser in einen Reaktor R eingespeist und einem Gemisch umgesetzt werden, das neben Polyoxymethylendimethylether auch noch Formaldehyd, Wasser, Methylenglykol, Polyoxymethylenglykole, Methanol, Hemiformale und Methylal enthält. Dieses Polyoxymethylendimethylether-haltige Gemisch wird in eine Destillationseinheit eingeleitet und in eine Leichtsieder- und Schwersiederfraktion aufgetrennt.

CN 104557483 A beschreibt die Herstellung von Polyformaldehyddimethylether aus Methanol, Methylal und Polyformaldehyd.

CN 102 372 615 B beschreibt beschreibt die Herstellung von Polyoxymethylendimethylether durch Umsetzung von Methylal und Trioxan in einer Reaktivdestillationseinheit.

US 2002/007089 A1 beschreibt ein Verfahren zur Herstellung sauerstoffhaltiger organischer Verbindungen, wobei ein Reaktantenstrom, der unter anderem Methanol und eine Formaldehydquelle enthält, in Gegenwart eines sauren Katalysators in einer Reaktivdestillationseinheit erwärmt wird, so dass ein Produktstrom erhalten wird, der Wasser, Methanol, Methylal und einen oder mehrere Polyoxymethylendimethylether enthält.

Eine Aufgabe der vorliegenden Erfindung ist die Herstellung von Polyoxymethylendimethylether (insbesondere solchen, die drei bis fünf Oxymethylen-Einheiten enthalten) über ein Verfahren, das möglichst einfach durchgeführt werden kann und eine hohe Effizienz aufweist.

Gelöst wird die Aufgabe durch ein Verfahren zur Herstellung von Polyoxymethylendimethylether, wobei als Edukte

(i) eine Formaldehydquelle und
(ii) mindestens eine Verbindung der Formel (I)

$$H_3C-O-R \qquad (I)$$

wobei
R: H oder $-(CH_2O)_x-CH_3$ mit x=0 oder 1

in eine Reaktivdestillationseinheit eingeleitet und zu Polyoxymethylendimethylether umgesetzt werden, wobei in dem Verfahren die Herstellung von Polyoxymethylendimethylether ausschließlich in der Reaktivdestillationseinheit erfolgt und die Reaktivdestillationseinheit eine oder mehrere Reaktionszonen, in denen jeweils ein Katalysator vorliegt, und eine oder mehrere destillative Trennzonen aufweist.

Im Rahmen der vorliegenden Erfindung wurde erkannt, dass sich die Formaldehydquelle und die Verbindung für die Methylverkappung (d.h. Verbindung (I)) in sehr effizienter Weise in einer Reaktivdestillationseinheit zu Polyoxymethylendimethylether umsetzen lassen, wobei eine im Wesentlichen vollständige Umsetzung des Formaldehyds erzielt werden kann. Da das in der Reaktivdestillationseinheit erhaltene Kopfprodukt (auch als Leichtsiederfraktion bezeichnet) relativ wenig Formaldehyd enthält oder sogar Formaldehyd-frei ist, kann eine effiziente Wasserabtrennung erfolgen. Im Vergleich zu einem Verfahren, das in einem ersten Schritt aus den Edukten die Polyoxymethylendimethylether herstellt und anschließend in einem zweiten Schritt das Reaktionsgemisch in die Fraktionen auftrennt, ist der einstufige Prozess der vorliegenden Erfindung thermodynamisch begünstigt. Da außerdem die Synthese der Polyoxymethylendimethylether ausschließlich in der Reaktivdestillationseinheit stattfindet, ist ein vorgeschalteter Reaktor für die OME-Synthese nicht erforderlich. Dies ermöglicht die Realisierung einer wesentlich kompakteren Anlage.

Die Formaldehydquelle ist beispielsweise Formaldehyd (z.B. als wässrige Formaldehydlösung (Formalin)), Trioxan oder Paraformaldehyd.

Für das Verfahren der vorliegenden Erfindung können beispielsweise kommerziell erhältliches Formaldehyd (z.B. als wässrige Formaldehydlösung (Formalin)) und kommerziell erhältliches Methylal verwendet werden. Alternativ ist es im Rahmen der vorliegenden Erfindung auch möglich, das Formaldehyd und das Methylal über eine oxidative Dehydrierung von Methanol zu erzeugen. Die Herstellung eines Gemisches, das Formaldehyd und Methylal enthält, durch eine oxidative Dehydrierung von Methanol wird in DE 10 2017 218 782 A1 beschrieben.

Das molare Verhältnis der in die Reaktivdestillationseinheit eingeleiteten Verbindung der Formel (I) und des Formaldehyds (d.h. n(Verbindung (I))/n(Formaldehyd)) kann im Rahmen der vorliegenden Erfindung über einen relativ breiten Bereich variiert werden und liegt beispielsweise im Bereich von 100/1 bis 1/10. Sofern die Formaldehydquelle Trioxan oder Paraformaldehyd ist, bezieht sich die molare Menge des Formaldehyds auf die in Trioxan oder Paraformaldehyd chemisch gebundene Formaldehydmenge (d.h. die molare Menge an -CH$_2$-O-Einheiten in diesen Verbindungen).

Das erfindungsgemäße Verfahren ermöglicht eine hohe Flexibilität hinsichtlich der Eduktzusammensetzung (hohe Feedstock-Flexibilität). Beispielsweise können die in die Reaktivdestillationseinheit eingeleiteten Edukte reich an Methylal oder reich an Methanol sein.

Die Reaktivdestillationseinheit (z.B. eine Reaktivdestillationskolonne) weist eine oder mehrere Reaktionszonen, in denen jeweils ein Katalysator (insbesondere ein Feststoff-Katalysator) vorliegt, und eine oder mehrere destillative Trennzonen auf. Die destillative Trennzone enthält beispielsweise Einbauten für die destillative Trennung, insbesondere Böden, Füllkörper oder strukturierte Packungen, wie sie dem Fachmann allgemein bekannt sind. Die Immobilisierung des Katalysators in den Reaktionszonen kann auf eine dem Fachmann bekannte Weise erfolgen, z.B. als regelloser Schüttfüllkörper; in Form von Katalysator-befüllten Drahtgewebekugeln oder als Katalysatorformkörper, die auf einem Boden in der Reaktionszone angebracht sind.

Sofern die Reaktivdestillationseinheit zwei oder mehr Reaktionszonen enthält, kann es bevorzugt sein, dass zwischen zwei Reaktionszonen jeweils eine destillative Trennzone vorliegt.

Katalysatoren für die Polyoxymethylendimethylether-Synthese sind dem Fachmann bekannt. Üblicherweise ist der Katalysator ein saurer Katalysator. Dabei können Feststoff-Katalysatoren oder auch flüssige Säuren verwendet werden. Beispielhaft können folgende Katalysatoren genannt werden: Ionenaustauscherharze, die saure Gruppen aufweisen (d.h. Kationenaustauscherharze), Zeolite, Aluminosilicate, Übergangsmetalloxide (die optional auf einem Trägermaterial vorliegen können), Graphenoxid, Mineralsäuren (z.B. Schwefelsäure), organische Säuren (z.B. HCOOH), saure ionische Flüssigkeiten, Oxoniumsalze (z.B. ein Trimethyloxoniumsalz).

Die Edukte können beispielsweise zwischen einer destillativen Trennzone und einer Reaktionszone in die Reaktivdestillationseinheit eingeleitet werden. Im Rahmen der vorliegenden Erfindung können die Edukte aber auch im Bereich der Reaktionszone oder der destillativen Trennzone in die Reaktivdestillationseinheit eingeleitet werden. Druck und Temperatur in der Reaktivdestillationseinheit sind bevorzugt so gewählt, dass die eingeleiteten Edukte teilweise oder vollständig in die Gasphase überführt werden. In der Reaktionszone werden die Edukte in Anwesenheit des Katalysators zu Polyoxymethylendimethylether umgesetzt. In der destillativen Trennzone erfolgt die Abtrennung des Formaldehyds, der Verbindung (I) (z.B. Methylal und/oder Methanol) und des Wassers von Polyoxymethylendimethylether. Es wird eine Schwersiederfraktion erhalten, in der Polyoxymethylendimethylether mit mindestens drei Oxymethylen-Einheiten (z.B. drei bis fünf Oxymethylen-Einheiten) vorliegen. Üblicherweise enthält die Schwersiederfraktion auch noch OME$_2$ und/oder optional OME$_{n>5}$. Weiterhin wird eine Leichtsiederfraktion erhalten, die Wasser und nicht umgesetzte Edukte enthält. Da mit dem erfindungsgemäßen Verfahren ein sehr hoher Umsetzungsgrad der Formaldehydquelle realisiert werden kann, enthält die Leichtsiederfraktion bevorzugt nur relativ geringe Mengen an Formaldehyd (z.B. weniger als 30 Vol% oder weniger als 20 Vol%) oder ist sogar Formaldehyd-frei. Die Leichtsiederfraktion enthält üblicherweise kein OME$_{n\geq4}$ und bevorzugt auch kein OME$_3$.

In einer bevorzugten Ausführungsform liegt in der Reaktionszone ein Hohlkörper vor, der den Katalysator zumindest teilweise umschließt. Der Hohlkörper kann von gasförmigen Edukten, die in der Reaktivdestillationseinheit aufsteigen, durchströmt werden (z.B. durch Öffnungen an seiner Unter- und Oberseite), verhindert aber im Wesentlichen einen Kontakt zwischen flüssigen Polyoxymethylendimethylether, die in der Reaktivdestillationseinheit nach unten zurückfließen, und dem Katalysator. Beispielsweise fließen die flüssigen Polyoxymethylendimethylether zumindest teilweise oder sogar ausschließlich seitlich an dem Hohlkörper vorbei. Der Hohlkörper kann beispielsweise rohr- oder glockenförmig sein. Andere Geometrien sind aber ebenfalls möglich. Der Hohlkörper kann beispielsweise aus einem Metall oder einer Keramik gefertigt sein. Ein in der Reaktivdestillationseinheit nach oben strömendes Gas dringt beispielsweise in den Behälter über eine Öffnung an dessen Unterseite ein und kann den Behälter über eine Öffnung an dessen Oberseite verlassen. Wenn ein flüssiger Polyoxymethylendimethylether in der Reaktivdestillationseinheit nach unten zurückfließt, kann er beispielsweise aufgrund des an der Oberseite des Hohlkörpers ausströmenden Gases nicht oder nur in geringem Maße in den Hohlkörper einfließen, sondern fließt seitlich an dem Hohlkörper vorbei. Durch diese Maßnahme wird die Rückreaktion von bereits gebildeten längerkettigen Polyoxymethylendimethylethern zu kürzerkettigen Verbindungen vermindert.

Die Reaktionszone kann beispielsweise zumindest teilweise von einer Membran umgeben sein. Die Membran ermöglicht die selektive Abtrennung bestimmter Komponenten aus der Reaktionszone. Bevorzugt ist die Membran eine wasserdurchlässige Membran, so dass über diese Membran Wasser von den anderen Komponenten in der Reaktionszone abgetrennt werden kann. Durch diese Maßnahme kann ein noch höherer Umsetzungsgrad der Edukte realisiert werden. Die Membran kann beispielsweise rohrförmig sein. Andere Geometrien sind aber ebenfalls möglich. Sofern die Reaktivdestillationseinheit zwei oder mehr Reaktionszonen enthält, kann die Membran beispielsweise in Form eines Rohres vorliegen, das mindestens zwei der Reaktionszonen umschließt. In dieser beispielhaften Ausführungsform liegt also in der Reaktivdestillationseinheit eine rohrförmige Membran vor, die zwei oder mehr Reaktionszonen (optional alle

anwesenden Reaktionszonen) umschließt. Geeignete Membrane, insbesondere solche für die Abtrennung von Wasser, sind dem Fachmann bekannt. Die Membran kann eine anorganische Membran oder eine Polymermembran sein. Die anorganische Membran ist beispielsweise eine Keramikmembran oder eine Zeolithmembran.

In der destillativen Trennzone kann optional ein basisches Material (z.B. ein basisches Ionenaustauscherharz) vorliegen. Sofern ein saures Katalysatormaterial von der Reaktionszone in die destillative Trennzone ausgetragen wird, kann es durch das in der destillativen Trennzone vorliegende basische Material neutralisiert werden.

Die destillative Trennzone ist bevorzugt Katalysator-frei, enthält also keinen Katalysator für die Polyoxymethylendimethylether-Synthese.

In dem erfindungsgemäßen Verfahren kann die Reaktivdestillationseinheit unter relativ milden Bedingungen betrieben werden, beispielsweise bei einem Druck im Bereich von 0,1 bis 10 bar, bevorzugter 0,2 bar bis 5 bar, noch bevorzugter 0,2 bar bis 1 bar; und einer Temperatur im Bereich von 20-300 °C, bevorzugter 50 °C bis 200 °C, noch bevorzugter 50 °C bis 150 °C.

Die Reaktivdestillationseinheit wird beispielsweise in der Reaktionszone mit einer massebezogenen Raumgeschwindigkeit WHSV (engl.: weight hourly space velocity) von 0,1-150 h$^{-1}$, bevorzugter 1-150 h$^{-1}$, noch bevorzugter 5-50 h$^{-1}$ betrieben. WHSV gibt das Verhältnis zwischen dem Eduktmassenstrom und der eingesetzten Katalysatormasse an.

Die Reaktivdestillationseinheit kann einen Verdampfer ("Reboiler") und einen Kondensator enthalten. Dies ist dem Fachmann grundsätzlich bekannt.

Die Schwersiederfraktion sammelt sich am Boden der Reaktivdestillationseinheit und wird daher auch als Sumpfprodukt oder Kolonnensumpf bezeichnet. Nach dem Abziehen aus der Reaktivdestillationseinheit kann die Schwersiederfraktion optional noch weiteren Destillationen (z.B. einer fraktionierten Destillation) unterzogen werden, um die gewünschten Polyoxymethylendimethylether-Fraktionen (insbesondere die OME$_{3-5}$-Fraktionen) zu isolieren.

In einer bevorzugten Ausführungsform wird die Leichtsiederfraktion in eine Trenneinheit eingeleitet, wobei ein wasserarmer oder wasserfreier Strom und ein wasserreicher Strom erhalten werden.

Die Trenneinheit kann sich beispielsweise außerhalb der Reaktivdestillationseinheit befinden. Alternativ ist es auch möglich, dass sich die Trenneinheit in der Reaktivdestillationseinheit, z.B. in deren Kopfbereich, befindet. Optional kann die Leichtsiederfraktion vor dem Einleiten in die Trenneinheit durch eine Kondensatoreinheit teilweise oder vollständig verflüssigt werden. Wie oben bereits erwähnt, werden in der Trenneinheit ein wasserarmer oder wasserfreier Strom und ein wasserreicher Strom erzeugt.

Die Abtrennung des Wassers in der Trenneinheit erfolgt beispielsweise durch eine Flüssig-Flüssig-Phasentrennung (bzw. eine Flüssig-Flüssig-Phasenentmischung), eine Pervaporation oder Dampfpermeation (z.B. unter Verwendung einer oder mehrerer Membrane), eine Adsorption, eine Absorption, eine thermische Phasentrennung oder eine Extraktion.

Bei der Flüssig-Flüssig-Phasenseparation werden z.B. eine Mischungslücke und Dichteunterschiede zwischen dem Wasser und den organischen Komponenten genutzt.

Geeignete Membrane für die Pervaporation oder Dampfpermeation sind dem Fachmann bekannt. Es können Polymermembrane oder auch anorganische Membrane verwendet werden. Geeignete Materialien für die Wasserabtrennung durch Adsorption sind dem Fachmann ebenfalls bekannt. Beispielhaft können in diesem Zusammenhang Zeolithe, Aktivkohle, Metalloxide, Kieselgele, Silikagele oder Salze genannt werden.

Im Rahmen der vorliegenden Erfindung kann die Trenneinheit thermisch mit dem Verdampfer ("Reboiler") und/oder dem Kondensator der Reaktivdestillationseinheit verbunden sein. Durch diese Maßnahme kann der Energiebedarf der Trenneinheit zumindest teilweise durch den Verdampfer und/oder den Kondensator gedeckt werden.

Der wasserarme oder wasserfreie Strom wird bevorzugt zumindest teilweise zu der Reaktivdestillationseinheit zurückgeführt. Die Rückführung kann beispielsweise erfolgen, indem der wasserarme oder wasserfreie Strom außerhalb der Reaktivdestillationseinheit den Edukten zugeführt und mit diesen in die Reaktivdestillationseinheit eingespeist wird. Zusätzlich oder alternativ kann der wasserarme oder wasserfreie Strom direkt in die Reaktivdestillationseinheit zurückgeführt werden, beispielsweise im Bereich der Reaktionszone der Reaktivdestillationseinheit. Durch eine Variation des Recyclingverhältnisses (d.h. des Anteils des wasserarmen oder wasserfreien Stroms, der in die Reaktivdestillationseinheit zurückgeführt wird) kann auch die Zusammensetzung des Endprodukts gesteuert werden.

Das erfindungsgemäße Verfahren weist eine sehr hohe Effizienz auf, beispielsweise mehr als 60%. Die Verfahrenseffizienz kann mit der folgenden Formel bestimmt werden:

$$\eta_{eff} = \frac{(\dot{m}_i \, LHV_i)_{product}}{(\dot{m}_i \, LHV_i)_{feed} + E_{process}}$$

wobei

$\eta_{eff}$      die Verfahrenseffizienz,

$\dot{m}_i$      der Massenstrom der Komponente i,

$LHV_i$      der unterer Heizwert der Komponente i und

$E_{process}$      der Energiebedarf des Verfahrens sind.

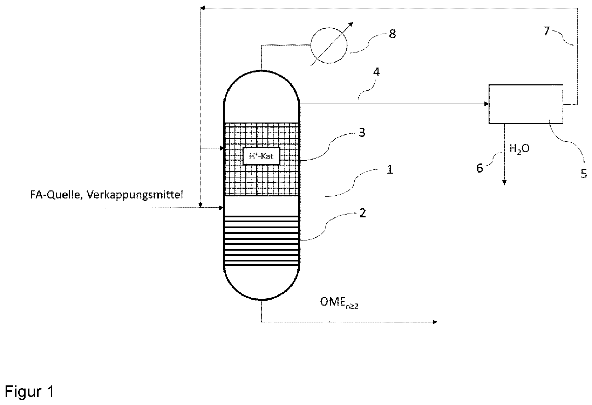

Die vorliegende Erfindung wird nun eingehender anhand einer in Figur 1 schematisch dargestellten beispielhaften Ausführungsform erläutert.

Die Reaktivdestillationseinheit 1 weist eine destillative Trennzone 2 und eine Reaktionszone 3 auf. Die destillative Trennzone enthält dem Fachmann bekannte Einbauten für die destillative Trennung, z.B. Böden, Füllkörper oder strukturierte Packungen. In der Reaktionszone 3 liegt ein saurer Katalysator vor, der die Umsetzung der Edukte zu Polyoxymethylendimethylether katalysiert.

Eine Formaldehydquelle ("FA-Quelle", z.B. Formaldehyd, Trioxan oder Paraformaldehyd) sowie ein Verkappungsmittel (d.h. eine Verbindung, die eine Hydroxygruppe in eine Methoxygruppe überführen kann) wie z.B. MeOH, Methylal oder Dimethylether werden als Edukte in die Reaktivdestillationseinheit 1 eingeleitet. Optional kann der Feed in die Reaktivdestillationseinheit 1 auch noch Wasser enthalten.

Die Einleitung des Eduktstroms in die Reaktivdestillationseinheit 1 erfolgt beispielsweise bei einer Temperatur und einem Druck, durch die die Edukte teilweise oder vollständig in die Gasphase überführt werden. Die Edukte steigen in die Katalysator-haltige Reaktionszone 3 auf und reagieren dort zu Polyoxymethylendimethylethern. Durch Kondensationsreaktionen kann sich in der Reaktionszone 3 auch Wasser bilden.

Die in der Reaktionszone 3 gebildeten Polyoxymethylendimethylether fließen nach unten und gelangen in die destillative Trennzone 2, in der eine Trennung der Polyoxymethylendimethylether von nicht umgesetzten Edukten und Wasser stattfindet.

Am Boden der Reaktivdestillationseinheit 1 sammelt sich die Schwersiederfraktion, die im Wesentlichen aus Polyoxymethylendimethylethern besteht. Die Schwersiederfraktion wird aus der Reaktivdestillationseinheit 1 abgezogen und kann gegebenenfalls weiteren Aufarbeitungsschritten (z.B. einer fraktionierten Destillation) unterzogen werden.

In der Reaktionszone 3 wird das Formaldehyd unter kontinuierlicher Bildung von Polyoxymethylendimethylether größtenteils oder sogar vollständig aufgebraucht. Die Leichtsiederfraktion, die als Kopfprodukt über Leitung 4 aus der Reaktivdestillationseinheit 1 abgezogen wird, enthält Wasser, ist aber im Wesentlichen Formaldehyd-frei. Neben Wasser enthält die Leichtsiederfraktion beispielsweise noch eine oder mehrere Verbindungen der Formel (I) (z.B. Methylal und/oder Methanol). Gegebenenfalls kann auch noch $OME_2$ in der Leichtsiederfraktion vorliegen.

Nach einer optionalen Verflüssigung durch einen Kondensator 8 wird die Leichtsiederfraktion einer Trenneinheit 5 zugeführt. In dieser Trenneinheit 5 wird ein wasserreicher Strom 6 sowie ein wasserarmer oder wasserfreier Strom 7 erzeugt. Der wasserarme bzw. wasserfreie Strom 7 wird dem Eduktstrom zugeführt und mit diesem in die Reaktivdestillationseinheit 1 eingeleitet. Der wasserarme oder wasserfreie Strom 7 kann auch direkt in die Reaktionszone 3 der Reaktivdestillationseinheit 1 zurückgeführt werden.

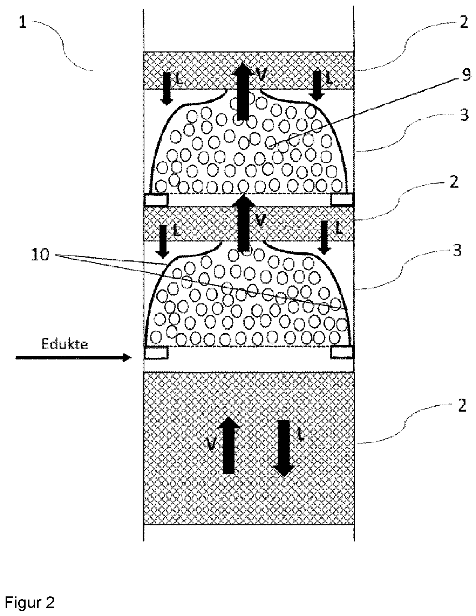

Eine weitere bevorzugte Ausführungsform der Erfindung wird in Figur 2 veranschaulicht.

Figur 2 zeigt einen Ausschnitt der Reaktivdestillationseinheit 1 mit destillativen Trennzonen 2 und Reaktionszonen 3. In den Reaktionszonen 3 befindet sich jeweils ein Katalysator 9 (z.B. ein saures Ionenaustauscherharz). Die Wandung eines Hohlkörpers 10 umschließt den Katalysator 9. An seiner Ober- und Unterseite ist der Hohlkörper 10 geöffnet. Nach oben strömende gasförmige Verbindungen V können den Hohlkörper 10 durchströmen, während flüssige Polyoxymethylendimethylether L, die in der Reaktivdestillationseinheit 1 nach unten zurückfließen, zumindest teilweise seitlich an dem Hohlkörper 10 vorbeifließen und daher mit dem Katalysator 9 nicht oder nur in geringem Maße in Kontakt kommen. Somit wird die Rückreaktion von bereits gebildeten längerkettigen Polyoxymethylendimethylethern zu kürzerkettigen Verbindungen vermindert.

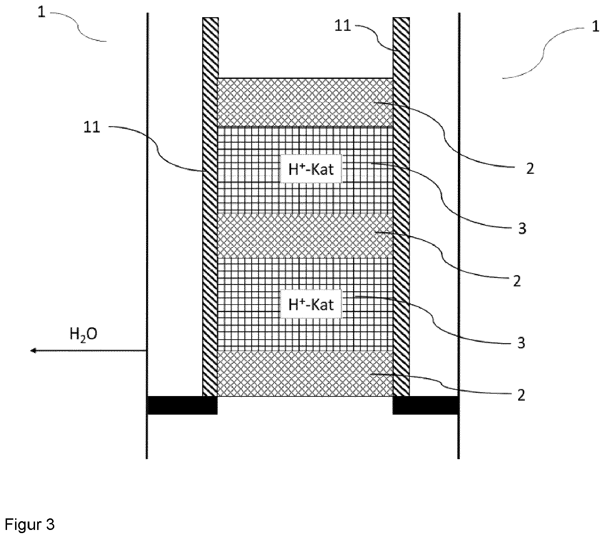

Eine weitere beispielhafte Ausgestaltung der Erfindung wird in Figur 3 veranschaulicht.

Figur 3 zeigt einen Ausschnitt der Reaktivdestillationseinheit 1 mit Reaktionszonen 3 (in denen jeweils ein saurer Katalysator ("H⁺-Kat") vorliegt) und destillativen Trennzonen 2. Die Reaktionszonen 3 sind von einer Membran 11, z.B. einer ZeolithMembran, umgeben. Über die wasserdurchlässige Membran 11 kann Wasser von den anderen Komponenten in den Reaktionszonen 3 abgetrennt und als wasserreicher Strom aus der Reaktivdestillationseinheit abgezogen werden. Die Membran 11 kann beispielsweise in Form eines Rohres, das eine oder mehrere der Reaktionszonen 3 umschließt, ausgestaltet sein.

**Patentansprüche**

1. Verfahren zur Herstellung von Polyoxymethylendimethylether, wobei als Edukte

    (i) eine Formaldehydquelle und
    (ii) mindestens eine Verbindung der Formel (I)

    $$H_3C\text{-}O\text{-}R \qquad (I)$$

    wobei
    R: H oder $\text{-}(CH_2O)_x\text{-}CH_3$ mit x=0 oder 1

    in eine Reaktivdestillationseinheit eingeleitet und zu Polyoxymethylendimethylether umgesetzt werden, wobei in dem Verfahren die Herstellung von Polyoxymethylendimethylether ausschließlich in der Reaktivdestillationseinheit erfolgt und die Reaktivdestillationseinheit eine oder mehrere Reaktionszonen, in denen jeweils ein Katalysator vorliegt, und eine oder mehrere destillative Trennzonen enthält.

2. Verfahren nach Anspruch 1, wobei der Katalysator in der Reaktivdestillationseinheit ein saurer Katalysator, insbesondere ein Ionenaustauscherharz, das saure Gruppen aufweist, ein Zeolith, ein Aluminosilicat, ein Übergangsmetalloxid, Graphenoxid, eine Mineralsäure, eine organische Säure, eine saure ionische Flüssigkeit oder ein Oxoniumsalz ist.

3. Verfahren nach Anspruch 1 oder 2, wobei in der Reaktionszone ein Hohlkörper vorliegt, der den Katalysator zumindest teilweise umschließt.

4. Verfahren nach Anspruch 3, wobei der Hohlkörper von gasförmigen Edukten, die in der Reaktivdestillationseinheit aufsteigen, durchströmt werden kann, aber einen Kontakt zwischen flüssigen Polyoxymethylendimethylether, die in der Reaktivdestillationseinheit nach unten zurückfließen, und dem Katalysator im Wesentlichen verhindert.

5. Verfahren nach Anspruch 3 oder 4, wobei flüssige Polyoxymethylendimethylether, die in der Reaktivdestillationseinheit nach unten zurückfließen, zumindest teilweise seitlich an dem Hohlkörper vorbeifließen.

6. Verfahren nach einem der vorstehenden Ansprüche, wobei die Reaktionszone zumindest teilweise von einer Membran, insbesondere einer wasserdurchlässigen Membran, umgeben ist.

7. Verfahren nach einem der vorstehenden Ansprüche, wobei die in die Reaktivdestillationseinheit eingeleiteten Edukte reich an Methylal oder reich an Methanol sind.

8. Verfahren nach einem der vorstehenden Ansprüche, wobei die Reaktivdestillationseinheit bei einem Druck im Bereich von 0,1 bis 10 bar und einer Temperatur im Bereich von 20-300 °C betrieben wird.

9. Verfahren nach einem der vorstehenden Ansprüche, wobei in der Reaktionszone eine massebezogene Raumgeschwindigkeit WHSV von 0,1-150 $h^{-1}$ vorliegt

10. Verfahren nach einem der vorstehenden Ansprüche, wobei in der destillativen Trennzone ein basisches Material vorliegt.

11. Verfahren nach einem der vorstehenden Ansprüche, wobei eine Leichtsiederfraktion, die Wasser und nicht umgesetzte Edukte enthält, in eine Trenneinheit eingeleitet wird unter Erhalt eines wasserarmen oder wasserfreien Stroms und eines wasserreichen Stroms.

12. Verfahren nach Anspruch 11, wobei die Abtrennung des Wassers in der Trenneinheit durch eine Flüssig-Flüssig-Phasentrennung, eine Pervaporation, eine Dampfpermeation, eine Adsorption, eine Absorption, eine thermische Phasentrennung oder eine Extraktion erfolgt.

13. Verfahren nach Anspruch 11 oder 12, wobei der Verdampfer und/oder der Kondensator der Reaktivdestillationseinheit thermisch mit der Trenneinheit verbunden ist/sind und einen Teil der Energie für das Betreiben der Trenneinheit liefern kann/können.

**14.** Verfahren nach einem der Ansprüche 11 bis 13, wobei der wasserarme oder wasserfreie Strom zu der Reaktivdestillationseinheit zurückgeführt wird.

**15.** Verfahren nach Anspruch 14, wobei der wasserarme oder wasserfreie Strom außerhalb der Reaktivdestillationseinheit den Edukten zugeführt und dann mit den Edukten in die Reaktivdestillationseinheit eingeleitet wird; und/oder wobei der wasserarme oder wasserfreie Strom direkt in die Reaktivdestillationseinheit, bevorzugt im Bereich der Reaktionszone, zurückgeführt wird.

**Claims**

**1.** Method for producing polyoxymethylene dimethyl ether by introducing

> (i) a formaldehyde source and
> (ii) at least one compound of the formula (I)

> $$H_3C-O-R \qquad (I)$$

> where

> R is H or $-(CH_2O)_x-CH_3$ with x being 0 or 1
> as reactants into a reactive distillation unit and reacting them to give polyoxymethylene dimethyl ether, wherein the method produces polyoxymethylene dimethyl ether exclusively in the reactive distillation unit and the reactive distillation unit comprises one or more reaction zones, each accommodating a catalyst, and one or more distillative separation zones.

**2.** Method according to Claim 1, wherein the catalyst in the reactive distillation unit is an acidic catalyst, more particularly an ion exchange resin containing acidic groups, a zeolite, an aluminosilicate, a transition metal oxide, graphene oxide, a mineral acid, an organic acid, an acidic ionic liquid or an oxonium salt.

**3.** Method according to Claim 1 or 2, wherein the reaction zone accommodates a hollow body which at least partly encloses the catalyst.

**4.** Method according to Claim 3, wherein gaseous reactants which are ascending in the reactive distillation unit are able to flow through the hollow body, but the hollow body substantially prevents contact between the catalyst and liquid polyoxymethylene dimethyl ethers which are running back downward in the reactive distillation unit.

**5.** Method according to Claim 3 or 4, wherein liquid polyoxymethylene dimethyl ethers which are running back downward in the reactive distillation unit run at least partly laterally past the hollow body.

**6.** Method according to any of the preceding claims, wherein the reaction zone is surrounded at least partly by a membrane, more particularly a water-permeable membrane.

**7.** Method according to any of the preceding claims, wherein the reactants introduced into the reactive distillation unit are rich in methylal or rich in methanol.

**8.** Method according to any of the preceding claims, wherein the reactive distillation unit is operated at a pressure in the range from 0.1 to 10 bar and a temperature in the range of 20-300°C.

**9.** Method according to any of the preceding claims, wherein the weight hourly space velocity WHSV in the reaction zone is 0.1-150 $h^{-1}$.

**10.** Method according to any of the preceding claims, wherein the distillative separation zone accommodates a basic material.

**11.** Method according to any of the preceding claims, wherein a low-boiler fraction comprising water and unconverted reactants is introduced into a separating unit to give a low-water or water-free stream and a water-rich stream.

**12.** Method according to Claim 11, wherein the removal of the water in the separating unit takes place by a liquid-liquid phase separation, a pervaporation, a vapor permeation, an adsorption, an absorption, a thermal phase separation or an extraction.

**13.** Method according to Claim 11 or 12, wherein the evaporator and/or the condenser of the reactive distillation unit are/is connected thermally to the separating unit and are/is able to supply a part of the energy for the operation of the separating unit.

**14.** Method according to any of Claims 11 to 13, wherein the low-water or water-free stream is returned to the reactive distillation unit.

**15.** Method according to Claim 14, wherein the low-water or water-free stream is fed to the reactants outside the reactive distillation unit and then introduced with the reactants into the reactive distillation unit; and/or wherein the low-water or water-free stream is returned directly into the reactive distillation unit, preferably in the region of the reaction zone.


**Revendications**

**1.** Procédé de production de l'éther diméthylique du polyoxyméthylène, dans lequel on introduit dans une unité de distillation réactive, en tant que réactifs

(i) une source de formaldéhyde et
(ii) au moins un composé de Formule (I)

$$H_3C\text{-}O\text{-}R \qquad (I)$$

dans laquelle
R : H ou -(CH$_2$O)$_x$-CH$_3$ ationvec x = 0 ou 1,

et on les fait réagir pour obtenir l'éther diméthylique du polyoxyméthylène, procédé dans lequel la production de l'éther diméthylique du polyoxyméthylène a lieu exclusivement dans l'unité de distillation réactive, et l'unité de distillation réactive contient une ou plusieurs zones de réaction, dans chacune desquelles se trouvent un catalyseur, ainsi qu'une ou plusieurs zones de séparation par distillation.

**2.** Procédé selon la revendication 1, dans lequel le catalyseur de l'unité de distillation réactive est un catalyseur acide, en particulier une résine échangeuse d'ions qui comprend des groupes acides, une zéolite, un aluminosilicate, un oxyde d'un métal de transition, un oxyde de graphène, un acide minéral, un acide organique, un liquide ionique acide ou un sel d'oxonium.

**3.** Procédé selon la revendication 1 ou 2, dans lequel un corps creux qui entoure au moins partiellement le catalyseur est présent dans la zone de réaction.

**4.** Procédé selon la revendication 3, dans lequel le corps creux peut être traversé par des réactifs gazeux qui montent dans l'unité de distillation réactive, mais empêche pour l'essentiel un contact entre les éthers diméthyliques du polyoxyméthylène liquides qui redescendent dans l'unité de distillation réactive et le catalyseur.

**5.** Procédé selon la revendication 3 ou 4, dans lequel les éthers diméthyliques du polyoxyméthylène liquides qui redescendent dans l'unité de distillation réactive passent au moins partiellement latéralement par rapport au corps creux.

**6.** Procédé selon l'une des revendications précédentes, dans lequel la zone de réaction est au moins partiellement entourée d'une membrane, en particulier d'une membrane perméable à l'eau.

**7.** Procédé selon l'une des revendications précédentes, dans lequel les réactifs introduits dans l'unité de distillation réactive sont riches en méthylal ou riches en méthanol.

**8.** Procédé selon l'une des revendications précédentes, dans lequel l'unité de distillation réactive est exploitée sous une pression dans la plage de 0,1 à 10 bar et à une température dans la plage de 20 à 300 °C.

9. Procédé selon l'une des revendications précédentes, dans lequel on a dans la zone de réaction une vitesse spatiale horaire massique WHSV de 0,1 à 150 h$^{-1}$.

10. Procédé selon l'une des revendications précédentes, dans lequel un matériau basique est présent dans la zone de séparation par distillation.

11. Procédé selon l'une des revendications précédentes, dans lequel on introduit dans une unité de séparation une fraction légère qui contient de l'eau et des réactifs n'ayant pas réagi, avec obtention d'un courant à teneur faible ou nulle en eau et d'un courant à haute teneur en eau.

12. Procédé selon la revendication 11, dans lequel la séparation de l'eau dans l'unité de séparation a lieu par une séparation des phases liquide-liquide, une pervaporation, une perméation de vapeur, une adsorption, une absorption, une séparation thermique des phases ou une extraction.

13. Procédé selon la revendication 11 ou 12, dans lequel l'évaporateur et/ou le condenseur de l'unité de distillation réactive sont thermiquement reliés à l'unité de séparation et peuvent fournir une partie de l'énergie nécessaire à l'exploitation de l'unité de séparation.

14. Procédé selon l'une des revendications 11 à 13, dans lequel le courant à teneur faible ou nulle en eau est renvoyé dans l'unité de distillation réactive.

15. Procédé selon la revendication 14, dans lequel le courant à teneur faible ou nulle en eau est amené aux réactifs à l'extérieur de l'unité de distillation réactive, puis est introduit avec les réactifs dans l'unité de séparation réactive ; et/ou dans lequel le courant à teneur faible ou nulle en eau est directement renvoyé dans l'unité de distillation réactive, de préférence dans le domaine de la zone de réaction.

Figur 1

Figur 2

Figur 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3323800 A1
- CN 104557483 A
- CN 102372615 B
- US 2002007089 A1
- DE 102017218782 A1

**In der Beschreibung aufgeführte Nicht-Patentliteratur**

- **M. OUDA et al.** *React. Chem. Eng.,* 2017, vol. 2, 50-59
- **M. OUDA et al.** *React. Chem. Eng.,* 2018, vol. 3, 676-695
- **Z. WANG et al.** *Applied Energy,* 2019, vol. 233-234, 599-611